# EUROPÄISCHE PATENTSCHRIFT

(11) **EP 3 846 673 B1**
(45) Veröffentlichungstag und Bekanntmachung des Hinweises auf die Patenterteilung: **06.07.2022**
(21) Anmeldenummer: 19761895.2
(22) Anmeldetag: 05.09.2019
(51) Int. Cl.: A61B 1/247, A61B 1/06, A61B 1/00

(54) **ZAHNÄRZTLICHES KAMERAHANDSTÜCK ZUM ERSTELLEN INTRAORALER AUFNAHMEN**
DENTAL CAMERA HANDPIECE FOR CREATING INTRAORAL PICTURES
PIÈCE À MAIN DE CAMÉRA DENTAIRE PERMETTANT DE RÉALISER DES ENREGISTREMENTS INTRABUCCAUX

(30) Priorität: 07.09.2018 EP 18193153
(43) Veröffentlichungstag der Anmeldung: 14.07.2021
(73) Patentinhaber: KaVo Dental GmbH, 88400 Biberach (DE)
(72) Erfinder: HECKENBERGER, Hans, 88433 Schemmerhofen (DE); LIEBERMANN, Bernd, 88212 Ravensburg (DE)
(74) Vertreter: Thun, Clemens
(86) Internationale Anmeldenummer: PCT/EP2019/073663
(87) Internationale Veröffentlichungsnummer: WO 2020/049083

(56) Entgegenhaltungen:
- DE-A1-102004 001 856
- US-A1- 2008 017 787
- US-A1- 2008 090 198
- US-A1- 2017 134 704
- US-A1- 2018 199 801

## Beschreibung

Die vorliegende Erfindung betrifft ein zahnärztliches Kamerahandstück, welches zum Erstellen intraoraler Aufnahmen vorgesehen ist.

Digitale zahnärztliche Kameras, mit deren Hilfe Aufnahmen von Zähnen oder anderen Objekten im Mundraum eines Patienten erstellt werden können, finden in der Zahnmedizin zunehmend Verwendung. Ein großer Vorteil derartiger Kameras besteht darin, dass die hiermit erstellten Aufnahmen unmittelbar beispielsweise auf einem Display eines zahnärztlichen Behandlungsplatzes dargestellt werden können und dementsprechend gegebenenfalls erforderliche Behandlungsmaßnahmen einem Patienten anschaulich erläutert werden können. Auch das Dokumentieren von Untersuchungs- und Behandlungsergebnissen wird mit Hilfe einer derartigen Intraoralkamera deutlich vereinfacht. Dabei können mit Hilfe moderner zahnärztlicher Kameras sowohl Einzelaufnahmen beziehungsweise Standbilder als auch Videos erstellt werden.

In klassischer Weise werden mit Hilfe der derzeit bekannten Intraoralkameras sogenannte Weißlichtaufnahmen erstellt. Das heißt, der von der Optik der Kamera erfasste Bereich wird mit einer Weißlichtquelle beleuchtet und das von der Kamera erfasste Farbbild setzt sich aus den Wellenlängen des sichtbaren Bereichs zusammen. Damit entspricht das von der Kamera erstellte Bild im Wesentlichen der Sichtweise eines menschlichen Beobachters, wobei das Platzieren der Kamera in unmittelbarer Nähe des zu betrachtenden Objekts, also beispielsweise eines Zahns, das Erkennen einzelner Details ermöglicht.

Neben dem Erstellen derartiger Weißlichtaufnahmen existieren in der Zahnmedizin allerdings auch weitere optische Diagnoseverfahren, mit deren Hilfe beispielsweise das frühzeitige Erkennen von Karies unterstützt werden soll.

Ein erstes bekanntes Diagnoseverfahren ist hierbei die sogenannte Transillumination, bei der ein zu untersuchender Zahn durchleuchtet wird und wiederum mit Hilfe einer Kamera erfasst wird, in welcher Weise der Zahn das in ihn eingekoppelte Licht streut. Dabei wird die Tatsache genutzt, das kariöses Zahngewebe Licht in anderer Weise streut als gesundes Zahngewebe. Diese Vorgehensweise ist somit vergleichbar zu einer klassischen Röntgenaufnahme eines Zahns, wobei allerdings im Vergleich zur Röntgenaufnahme bei der Transillumination Licht einer deutlich längeren Wellenlänge verwendet wird und dementsprechend die zum Durchleuchten des Zahns verwendete Strahlung weniger problematisch im Hinblick auf eventuelle gesundheitliche Nebenwirkungen ist.

Ein entsprechendes zahnärztliches System zum Transilluminieren von Zähnen ist beispielsweise aus der EP 2 452 614 A1 der Anmelderin bekannt. Es hat sich hierbei herausgestellt, dass die Qualität der im Rahmen eines derartigen Transilluminationsverfahrens erhaltenen Aufnahmen dann besonders gut ist, wenn der Zahn nicht mit sichtbaren Licht, sondern stattdessen mit Licht im nahen Infrarotbereich durchleuchtet wird und auch diese gestreute, hinsichtlich ihrer Wellenlänge im Wesentlichen nicht veränderte Infrarotstrahlung erfasst wird.

Ein weiteres, zum Erkennen von Karies genutztes optisches Verfahren beruht auf einer sogenannten Fluoreszenzdiagnose. Diese Fluoreszenzdiagnose beruht auf der Erkenntnis, dass kariöses Zahngewebe beim Beleuchten mit einer Anregungsstrahlung geeigneter Wellenlänge fluoresziert und Licht in Form von Fluoreszenzstrahlung abgibt. Wird also ein Zahn mit einer entsprechenden Anregungsstrahlung großflächig beleuchtet, so werden diejenigen Bereiche verstärkt Fluoreszenzstrahlung abgeben, in denen Karies vorhanden ist.

Entsprechende Fluoreszenzdiagnose-Verfahren sind im Stand der Technik vielfach und seit langer Zeit bekannt, wobei sich herausgestellt hat, dass dieses Verfahren insbesondere bei der Verwendung einer Anregungsstrahlung aus dem ultravioletten Bereich zu guten Ergebnissen führt. Die als Antwort hierauf entstehende Fluoreszenzstrahlung weist den allerdings eine geringere Energie, also eine etwas längere Wellenlänge auf und besteht dementsprechend aus kurzwelligem Licht im sichtbaren Bereich.

Während im Rahmen des oben erläuterten Transilluminationsverfahrens primär tieferliegende Karies aufweisende Bereiche erkannt werden können, kann mit Hilfe des Fluoreszenzdiagnose-Verfahrens in erster Linie an der Zahnoberfläche beziehungsweise Außenseite eines Zahns vorhandenes kariöses Gewebe erkannt werden. Beide Vorgehensweisen ergänzen sich also zu einem gewissen Grad, so dass eine Kombination beider Verfahren zur Optimierung einer berührungslosen Kariesdiagnose von Vorteil wäre.

Im Stand der Technik wird bereits vorgeschlagen, zwei der oben beschriebenen Diagnose-Verfahren innerhalb eines Geräts, also in einer Kamera zu realisieren. Die Erstellung einer Weißlichtaufnahme wird hier ebenfalls als Diagnose-Verfahren angesehen, sodass also grundsätzlich drei Diagnose-Verfahren beziehungsweise Aufnahmemodi denkbar sind, das Erstellen einer Weißlichtaufnahme, das Erstellen einer Transilluminationsaufnahme sowie das Erstellen einer Aufnahme im Rahmen einer Fluoreszenzdiagnose.

In diesem Zusammenhang wird beispielsweise in der DE 10 2006 041 020 A1 vorgeschlagen, die Transillumination eines Zahns mit einer Fluoreszenzdiagnose zu kombinieren. Die DE 100 43 749 A1 wiederum beschreibt das parallele Erfassen eines optischen Bilds im Rahmen einer Weißlichtaufnahme sowie zusätzlich auch eines Fluoreszenzbilds. In der DE 10 2013 006 636 A1 wiederum wird die Kombination des Erstellens eines optischen Bildes mit einem Bild basierend auf einem Transilluminationsverfahren oder einem Fluoreszenzdiagnoseverfahren vorgeschlagen. Die US 2008/090198 A1 offenbart ein zahnärztliches Kamerahandstück mit einem gemeinsamen Filter für Fluoreszenz und Weißlichtaufnahme.

Grundsätzlich wäre die Kombination aller drei Diagnose-Verfahren im Rahmen einer einzigen intraoralen Kamera wünschenswert, da - wie erläutert - alle Verfahren jeweils unterschiedliche Informationen hinsichtlich eines zu untersuchenden Zahns liefern. Es hat sich allerdings gezeigt, dass das gleichzeitige Realisieren aller drei Aufnahmemodi in einem Kamerahandstück nicht ohne weiteres durchführbar ist. Grund hierfür ist zunächst, dass bei jeder Aufnahmeart der zu untersuchende Zahn mit Licht einer anderen spektralen Zusammensetzung zu beleuchten ist. Entscheidendes Problem ist allerdings, dass abhängig vom jeweiligen Aufnahmemodus vorzugsweise Licht nur eines bestimmten Wellenlängenbereichs erfasst werden sollte. Die hierbei jeweils bevorzugten Bereiche grenzen teilweise nahe einander an, sodass beispielsweise das Erfassen eines Bildes für eine Weißlichtaufnahme sowie eines Bildes für eine Fluoreszenzdiagnose mit Hilfe eines einzigen optischen Systems nicht ohne weiteres möglich ist.

Zwar ist in diesem Zusammenhang bekannt, je nach Aufnahmeart entsprechende Filter einzusetzen, die dem Bildsensor vorgeschaltet sind, dies bedeutet jedoch, dass im optischen System der Kamera entsprechend verstellbare bzw. auswechselbare Filterkomponenten vorhanden sein müssen. Dies führt nicht nur zu einem komplexeren mechanischen Aufbau der Kamera, sondern kann auch zu einer erhöhten Fehleranfälligkeit des optischen Systems führen. Weiterhin ist dann jeweils bei einem Wechsel zwischen zwei verschiedenen Aufnahmemodi ein entsprechender Wechsel des Filters erforderlich, was einen schnellen Wechsel zwischen zwei verschiedenen Betriebsarten der Kamera verzögert. Allerdings wäre es von Vorteil, wenn Aufnahmen in verschiedenen Aufnahmemodi möglichst unmittelbar nacheinander durchgeführt werden können, da in diesem Fall sichergestellt ist, dass die Kamera den im Wesentlichen identischen Bildbereich erfasst und dementsprechend Aufnahmen unterschiedlicher Betriebsarten gut miteinander verglichen beziehungsweise miteinander kombiniert werden können.

Der vorliegenden Erfindung liegt deshalb die Aufgabenstellung zugrunde, ein zahnärztliches Kamerahandstück zum Erstellen intraoraler Aufnahmen zur Verfügung zu stellen, bei dem das optische System möglichst einfach aufgebaut ist und ein schneller Wechsel zwischen verschiedenen Aufnahmemodi ermöglicht wird.

Die Aufgabe wird durch ein zahnärztliches Kamerahandstück mit den Merkmalen des Anspruchs 1 gelöst. Vorteilshafte Weiterbildungen der Erfindung sind Gegenstand der abhängigen Ansprüche.

Der erfindungsgemäßen Lösung liegt die Erkenntnis zugrunde, dass das Erstellen von Bildern in den drei oben geschilderten unterschiedlichen Aufnahmemodi mit einem einzigen Filter durchgeführt werden kann, sofern dieser Filter in geeigneter Weise ausgestaltet ist. Insbesondere wird erfindungsgemäß vorgeschlagen, einen Filter zu verwenden, dessen Transmissionsspektrum einen ersten Bandpass aufweist, der im Wesentlichen dem Wellenlängenbereich des sichtbaren Lichts entspricht, sowie einen zweiten Bandpass, dessen Wellenlängen im Infrarotbereich liegen, wobei der erste Bandpass von dem zweiten Bandpass durch einen Sperrbereich getrennt ist, in dem das Filter im Wesentlichen dessen Wellenlängenbereich sperrt.

Gemäß der vorliegenden Erfindung wird also ein zahnärztliches Kamerahandstück zum Erstellen intraoraler Aufnahmen vorgeschlagen, welches aufweist:
- Beleuchtungsmittel zum Beleuchten eines vor einem Eintrittsfenster des Kamerahandstücks befindlichen Objekts,
- ein optisches System zum Abbilden des Objekts auf einen Bildsensor,
- einen digitalen Bildsensor zum Erfassen des mit Hilfe des optischen Systems auf den Sensor abgebildeten Objekts,
wobei die Beleuchtungsmittel dazu ausgebildet sind, das vor dem Eintrittsfenster des Kamerahandstücks befindliche Objekt entsprechend drei verschiedener Aufnahmemodi zu beleuchten, wobei die spektrale Zusammensetzung des von den Beleuchtungsmitteln abgegebenen Lichts für jeden Aufnahmemodus unterschiedlich ist, und
wobei das optische System einen dem Bildsensor vorgeschalteten und für alle drei Aufnahmemodi gemeinsam genutzten Filter aufweist, dessen Transmissionsspektrum einen ersten Bandpass aufweist, der im Wesentlichen dem Wellenlängenbereich des sichtbaren Lichts entspricht, sowie einen zweiten Bandpass, dessen Wellenlängen im Infrarotbereich liegen, wobei der erste Bandpass von dem zweiten Bandpass durch einen Sperrbereich getrennt ist, in dem der Filter im Wesentlichen dessen Wellenlängenbereich sperrt.

Dadurch, dass erfindungsgemäß einheitlich für alle drei Aufnahmemodi ein gemeinsamer Filter verwendet wird, muss zum Wechseln des Aufnahmemodus ausschließlich die durch die Kamera realisierte Beleuchtung angepasst werden. Dies kann allerdings durch eine entsprechend geeignete Ansteuerung der Beleuchtungsmittel sehr schnell durchgeführt werden, sodass nahezu ohne Zeitverzögerung aufeinander folgende Aufnahmen in unterschiedlichen Aufnahmemodi erstellt werden können. Dies hat zur Folge, dass die Aufnahmen den im Wesentlichen identischen Bildbereich erfassen und somit ohne großen zusätzlichen Aufwand überlagert beziehungsweise kombiniert werden können. Dies stellt einen extremen Vorteil gegenüber bislang bekannten Lösungen dar, da hierdurch in einfacher und komfortabler Weise eine umfangreiche, berührungslose Kariesdiagnose an einem Zahn durchgeführt werden kann.

Gemäß einer vorteilhaften Weiterbildung der Erfindung weisen hierbei die Beleuchtungsmittel eine erste Lichtquelle auf, welche dazu ausgebildet ist, in einem ersten Aufnahmemodus das Objekt mit sichtbarem Licht, insbesondere mit Weißlicht zu beleuchten. Diese erste Lichtquelle wird also immer dann aktiviert, wenn eine klassische Weißlichtaufnahme mit Hilfe der Kamera erstellt werden soll.

Weiterhin weisen die Beleuchtungsmittel vorzugsweise eine zweite Lichtquelle auf, welche dazu ausgebildet ist, in einem zweiten Aufnahmemodus, dem Fluoreszenzdiagnose-Modus, das Objekt mit einer Anregungsstrahlung zu beleuchten, deren spektrale Zusammensetzung im Wesentlichen unterhalb des sichtbaren Lichts liegt. Dieses im Ultravioletten liegende Licht wird dann also auf den Zahn gerichtet, um kariöse Bereiche anzuregen, eine entsprechende Fluoreszenzstrahlung abzugeben. Dabei ist vorzugsweise im Transmissionsspektrum des Filters der Wellenlängenbereich der Anregungsstrahlung von dem ersten Bandpass des Filters, der im Wesentlichen dem sichtbaren Licht entspricht, durch einen zweiten Sperrbereich getrennt, in dem der Filter im Wesentlichen dessen Wellenlängenbereich sperrt. Von dem Bildsensor wird also lediglich die in Reaktion auf die Anregungsstrahlung entstehende Fluoreszenzstrahlung erfasst, die Anregungsstrahlung selbst sowie im dazwischenliegenden Wellenlängenbereich liegende, im Rahmen von Lichtstreuung oder anderweitig entstehende Strahlung hingegen wird durch den Filter geblockt.

Dabei kann vorteilhaft ferner vorgesehen sein, dass zur Verbesserung der Bildqualität für den Fluoreszenzdiagnose-Modus beim Erstellen einer entsprechenden Aufnahme die Empfindlichkeitswerte für diejenigen Pixel des Bildsensors, welche den Blauanteil detektieren, verringert wird. Hierdurch wird die kurzwellige Anregungsstrahlung, welche beispielsweise auf Grund von evtl. Reflexionen trotz des Filters den Sensor erreicht, zusätzlich unterdrückt bzw. blockiert.

Gemäß einer anderen vorteilhaften Weiterbildung kann ferner vorgesehen sein, die Abgabe der Anregungsstrahlung zeitlich zu modulieren. Diese Maßnahme kann bei entsprechender Auswertung des von dem Bildsensor erfassten Bildes gegebenenfalls dazu herangezogen werden, nochmals besser in Antwort auf die Anregungsstrahlung entstehende Fluoreszenzstrahlung zu erkennen, da der Einfluss evtl. vorhandenen Umgebungslichts hierdurch minimiert werden kann.

Vorzugweise weisen die Beleuchtungsmittel ferner eine dritte Lichtquelle auf, welche dazu ausgebildet ist, in einem dritten Aufnahmemodus das Objekt mit Licht eines Wellenlängenbereichs zu beleuchten, der im Wesentlichen in dem zweiten Bandpass des Filters liegt. Diese dritte Lichtquelle dient der eingangs erläuterten Transillumination des Zahns, die vorzugsweise im Infrarotbereich des Lichts durchgeführt wird. Hier ist wesentlich, dass durch den Sensor primär das von dem Zahn gestreute Infrarotlicht erfasst wird, was dadurch sichergestellt wird, dass der zweite Bandpass mit dem Spektrum des im dritten Aufnahmemodus abgegebenen Lichts übereinstimmt. Durch den zwischen dem ersten Bandpass, also dem sichtbaren Licht, und dem zweiten Bandpass liegenden Sperrbereich wird ferner verhindert, dass langwelliges sichtbares Licht die Qualität der Transilluminationsaufnahme negativ beeinträchtigt.

Dabei ist die dritte Lichtquelle vorzugsweise Bestandteil eines Aufsatzes, der lösbar am vorderen Ende des Kamerahandstücks angeordnet ist, wobei in diesem Fall das Objekt, also beispielsweise der Zahn vorzugsweise in einer Richtung beleuchtet wird, die im Wesentlichen senkrecht zur Beleuchtungsrichtung für einen ersten oder zweiten Aufnahmemodus ist. Während für eine Weißlichtaufnahme oder eine Aufnahme im Rahmen der Fluoreszenzdiagnose das Beleuchten des Zahns vorzugsweise aus der Richtung erfolgt, aus der dieser auch dann wieder von der Kamera beobachtet wird, ist im Vergleich hierzu eine andere, insbesondere seitliche Einkopplung des Lichts in den Zahn für die Transillumination von Vorteil, da hier in erster Linie das Streulicht und kein von der Zahnoberfläche reflektiertes Licht erfasst werden soll.

Hierbei kann vorgesehen sein, dass mehrere Aufsätze zur Verfügung stehen, welche wahlweise am vorderen Ende des Kamerahandstücks angeordnet werden können, wobei die Aufsätze unterschiedlich gestaltete Lichtabstrahlelemente aufweisen, über welche das von der Lichtquelle erzeugte Licht abgegeben wird. Je nach Art des zu untersuchenden Zahns kann hierbei eine für die Transillumination optimale Lichteinkopplung in den Zahn realisiert werden.

Dabei weist das erfindungsgemäße Kamerahandstück ferner vorzugsweise einen Sensor auf, mit dessen Hilfe erkannt wird, ob ein entsprechender Aufsatz am vorderen Ende des Kamerahandstücks angeordnet ist, wobei gegebenenfalls auch der Typ des Aufsatzes durch den Sensor erkannt wird. Ferner sind vorzugsweise Mittel zur Energieversorgung der in einem Aufsatz befindlichen Lichtquelle vorgesehen, wobei diese Mittel insbesondere für eine induktive, also eine berührungslose Energieversorgung ausgebildet sind.

Zum Einhalten entsprechender Hygienevorschriften kann ferner vorgesehen sein, dass das Kamerahandstück eine sog. Hygieneschutzhülle aufweist, welche über das Handstück überstülpbar ist. Diese Hülle ist dann im Bereich des Eintrittsfensters lichtdurchlässig ausgebildet und liegt derart an diesem an, dass sie die Bilderfassung nicht negativ beeinträchtigt.

Vorzugsweise ist das Kamerahandstück dazu ausgebildet, die Beleuchtungsmittel entsprechend einer vorgegebenen Sequenz zu betreiben, welche der alternierenden Nutzung der verschiedenen Aufnahmemodi entspricht, wobei insbesondere eine Aufnahmesequenz die aufeinanderfolgende Aufnahme jeweils eines Bildes in jedem der drei Aufnahmemodi vorsieht. Da die erfindungsgemäße Lösung einen extrem schnellen Wechsel zwischen den verschiedenen Aufnahmemodi ermöglicht, können somit hinsichtlich ihres Bildausschnitts nahezu identische Aufnahmen in den verschiedenen Modi erstellt werden. Dies geschieht, ohne dass ein Benutzer des Kamerahandstücks dieses in spezieller Weise betätigen muss, so dass in sehr komfortabler Weise eine optimale optische Kariesdiagnose durchgeführt werden kann.

Hinsichtlich seines Aufbaus ist das Kamerahandstück vorzugsweise derart gestaltet, dass es eine längliche und rohrartig ausgestaltete Griffhülse aufweist, welche am vorderen Ende ein Lichteintrittsfenster aufweist, wobei das optische System zum Abbilden des Objekts auf den Bildsensor sowie der Bildsensor innerhalb der Griffhülse angeordnet sind und das optische System das über das Lichteintrittsfenster eintretende Licht auf den Bildsensor richtet. Dabei ist vorzugsweise eine Flächennormale des Lichteintrittsfensters im Wesentlichen senkrecht zur Längsachse der Griffhülse ausgerichtet, wobei dann im vorderen Endbereich der Griffhülse ein Umlenkelement, beispielsweise ein Spiegel oder ein Prisma, angeordnet ist.

Nachfolgend soll die Erfindung anhand der beiliegenden Zeichnungen näher erläutert werden. Es zeigen:
Figur 1 eine perspektivische Ansicht eines erfindungsgemäßen zahnärztlichen Kamerahandstücks;
Figur 2 einen Teilschnitt des vorderen Endbereichs des Kamerahandstücks ohne einen Aufsatz für die Transilluminationsaufnahme;
Figur 3 schematisch eine erste Variante des Aufbaus eines erfindungsgemäßen Kamerahandstücks;
Figur 4 das Transmissionsspektrum des erfindungsgemäßen zum Einsatz kommenden Filters;
Figur 5 die Sequenz einer Bildaufnahme zum aufeinanderfolgenden Erstellen von Bildern in drei verschiedenen Aufnahmemodi, und
Figur 6 eine zweite Variante eines Aufbaus eines erfindungsgemäßen Kamerahandstücks.

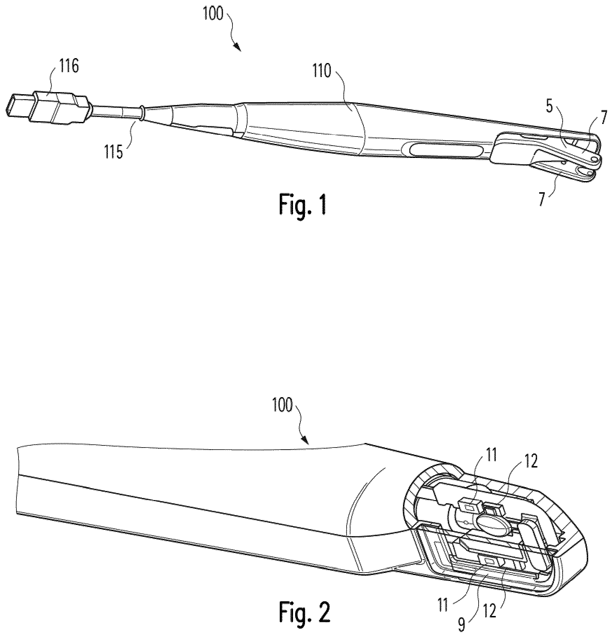

Figur 1 zeigt zunächst eine perspektivische Ansicht eines erfindungsgemäßen, allgemein mit dem Bezugszeichen 100 versehenen Kamerahandstücks. Dieses gleicht hinsichtlich seines Aufbaus grundsätzlich dem bspw. in der EP 2 452 614 A1 der Anmelderin beschriebenen Kamerahandstück. Es weist also zunächst eine längliche Griffhülse 110 auf, welche alle wesentlichen Komponenten zur Bildaufnahme beinhaltet und an ihrem rückseitigen Ende über ein Anschlusskabel 115 mit einem externen Gerät verbindbar ist. Wie in Figur 1 erkennbar, erfolgt vorzugsweise die Anbindung des Kamerahandstücks 100 mit Hilfe eines USB-Anschlusses 116 an einen PC. Bei diesem PC kann es sich insbesondere um einen PC eines zahnärztlichen Behandlungsplatzes oder einen sog. Hinterkopf-PC handeln. Mit Hilfe des Kamerahandstücks 100 erstellte Aufnahmen können dann unmittelbar auf einem Bildschirm des Behandlungsplatzes einem Patienten gezeigt werden. Dies erleichtert oftmals das Erläutern einer entsprechenden Diagnose sowie der sich hieraus ergebenden, durchzuführenden Behandlungsmaßnahmen. Alternativ zu einer Verbindung mit Hilfe eines USB-Anschlusses kann selbstverständlich auch eine anderweitige Verbindung des Kamerahandstücks 100 an ein externes Gerät erfolgen. Beispielsweise kann dieses auch dauerhaft mit einer zahnärztlichen Behandlungseinheit verbunden sein.

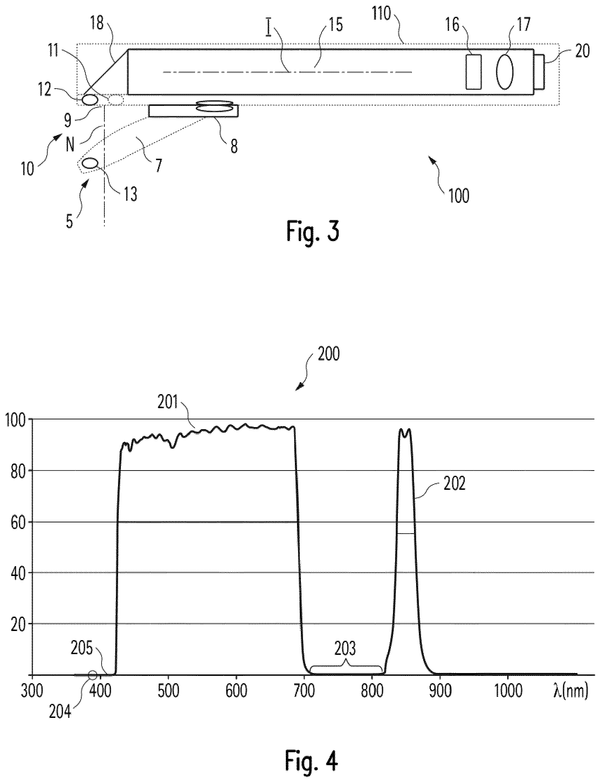

Die Griffhülse 110 des Kamerahandstücks 100 ist länglich ausgeführt, wobei die Bilderfassung am vorderen Ende der Griffhülse 110 erfolgt. Hierzu weist das Handstück 100 am vorderen Endbereich ein seitliches Lichteintrittsfenster 9 auf, welches auf das zu beobachtende Objekt, also bspw. den zu erfassenden Zahn oder Mundbereich des Patienten auszurichten ist. Die Flächennormale N (s. Figur 3) des Lichteintrittsfensters 9 ist somit im Wesentlichen senkrecht zur Längsachse I des Kamerahandstücks 100 ausgerichtet. Es hat sich gezeigt, dass bei einer derartigen Ausgestaltung die Handhabung des Kamerahandstücks 100 beim Erstellen intraoraler Aufnahmen erleichtert wird. Insbesondere ergibt sich eine ergonomisch deutlich günstigere Haltung, wenn die dem Mundraum zugewandten Zahnoberflächen oder die Kauflächen von Zähnen beobachtet werden sollen.

Das Lichteintrittsfenster 9 ist hierbei insbesondere im Teilschnitt von Figur 2 erkennbar, da hier ein am vorderen Ende des Kamerahandstücks 100 vorgesehener Aufsatz 5 nicht dargestellt ist bzw. das Kamerahandstück 100 in einem Zustand gezeigt ist, in dem dieser Aufsatz 5 entfernt wurde. Dieser Aufsatz 5 ist beim dargestellten Ausführungsbeispiel insbesondere für das Erstellen von Transilluminationsaufnahmen vorgesehen. Er weist - wie in Figur 1 erkennbar - zwei seitliche Arme 7 auf, an deren vorderen Enden jeweils später beschriebene Lichtquellen angeordnet sind. Beim Erstellen einer Transilluminationsaufnahme ist das Kamerahandstück 100 derart zu positionieren, dass das Sichtfenster 9 bspw. auf die Kaufläche eines zu beobachtenden Zahns ausgerichtet ist. Die Arme 7 mit den Lichtquellen sind dann jeweils zu beiden Seiten des zu untersuchenden Zahns angeordnet und deren Lichtquellen derart ausgerichtet, dass das für die Transillumination verwendete Licht seitlich in den Zahn eingekoppelt und durch diesen gestreut wird. Die Lichtquellen befinden sich hierbei jeweils anliegend an den unteren Seitenwänden das Zahn oder sogar im Bereich des Zahnfleisches, um das Licht möglichst homogen in den Zahn einzukoppeln. Diese beschriebene Anordnung der Komponenten zur Beleuchtung und zur Beobachtung des Zahns führt nicht nur zu einer sehr effizienten Einkopplung der für die Transillumination vorgesehenen Strahlung, sondern verhindert ferner auch, dass die Transilluminationsaufnahme durch auftretendes Streulicht negativ beeinträchtigt wird. Entsprechende Aufsätze sind detailliert in der EP 2 452 614 A1 beschrieben.

Eine derartige seitliche Lichteinkopplung ist also in erster Linie für die erwähnten Transilluminationsaufnahmen von Vorteil. Beim Erstellen einer Weißlichtaufnahme oder eine Aufnahme im Rahmen einer Fluoreszenzdiagnose hingegen ist keine seitliche Lichteinkopplung vorgesehen, sondern das Licht wird vorzugsweise aus Richtung des Sichtfensters 9 auf den Zahn bzw. allgemein auf das zu untersuchende Objekt gerichtet. Damit wird also bei der Transillumination eine im wesentlichen senkrechte Beleuchtungsrichtung im Vergleich zur Beleuchtungsrichtung für die beiden anderen Aufnahmemodi realisiert.

Die Tatsache, dass der Aufsatz 5 nur für einen der drei Aufnahmemodi benötigt wird, bedeutet, dass unter gewissen Umständen auch auf diesen verzichtet werden kann, was dann ggf. wiederum eine etwas flexiblere Positionierung der Kamera 100 für die weiteren Aufnahmearten ermöglicht. Vorzugsweise ist deshalb der Aufsatz 5 derart ausgestaltet, dass er lösbar auf das vordere Ende des Kamerahandstücks 100 aufgesetzt werden und bspw. mit diesem verrastet werden kann. Dabei kann ferner auch vorgesehen sein, dass unterschiedlich gestaltete Aufsätze 5 zur Verfügung stehen, die abhängig davon zum Einsatz kommen, welcher Typ eines Zahns mit Hilfe des Kamerahandstücks 100 erfasst werden soll. Auch in diesem Zusammenhang wird auf die EP 2 452 614 A1 verwiesen, in der mehrere unterschiedlich gestaltete entsprechende Aufsätze beschrieben sind. Diese können in vergleichbarer Weise auch bei dem vorliegenden erfindungsgemäßen Kamerahandstück 100 zum Einsatz kommen. Ferner kann auch vorgesehen sein, dass verschiedene Aufsätze 5 zur Verfügung stehen, die sich hinsichtlich der Länge bzw. Größe der Arme 7 unterscheiden, so dass abhängig von dem Alter des Patienten und damit der Größe der zu untersuchenden Zähne jeweils optimal geeignete Aufsätze 7 ausgewählt werden können.

Nachdem bislang in erster Linie der grundsätzliche Aufbau des Kamerahandstücks 100 beschrieben wurde, sollen nachfolgend die für die Bildaufnahme erforderlichen Komponenten anhand von Figur 3 erläutert werden. Gezeigt ist hierbei eine schematische Darstellung der Ausgestaltung des Handstücks 100, wobei vereinfacht diejenigen Bestandteile gezeigt sind, die für das Erstellen einer Aufnahme in den verschiedenen Aufnahmemodi verantwortlich sind.

Hierbei ist zunächst wie oben beschrieben erforderlich, dass abhängig von dem gewählten Aufnahmemodus das zu untersuchende Objekt in geeigneter Weise beleuchtet wird. Das Kamerahandstück 100 weist dementsprechend drei verschieden gestaltete Lichtquellen bzw. Leuchtmittel auf, die je nach Aufnahmemodus aktiviert sind.

Es handelt sich hierbei zunächst um eine erste Lichtquelle 11 zur Nutzung während eines Weißlicht-Aufhahmemodus. Diese Lichtquelle 11 soll also primär das zu untersuchende Objekt mit Licht aus dem sichtbaren Wellenlängenbereich beleuchten, wobei die spektrale Zusammensetzung des Lichts derart ist, dass sich insgesamt weißes Mischlicht ergibt. Denkbar wäre also insbesondere die Verwendung einer Kombination verschiedenfarbiger LEDs oder einer blauen LED, deren Licht durch geeignete Phosphore in Weißlicht konvertiert wird. Wichtig ist, dass es sich bei den Lichtquellen für die drei Aufnahmemodi nicht jeweils um Einzellichtquellen handeln muss, sondern dass diese durchaus wiederum durch eine Kombination mehrere Lichtquellen gebildet sein können.

Für die Fluoreszenzdiagnose wird im Gegensatz zur Weißlichtaufnahme die zweite Lichtquelle 12 benutzt, die den zu untersuchenden Zahn mit einer nicht sichtbaren, kurzwelligen Anregungsstrahlung im Bereich von etwa 400nm beleuchtet. Licht einer derartigen Wellenlänge veranlasst kariöses Zahngewebe, eine Fluoreszenzstrahlung im Bereich oberhalb von 480nm, also im orange-roten Lichtspektrum des sichtbaren Bereichs abzugeben. Dabei werden in der Literatur unterschiedliche Wellenlängen für die Anregungsstrahlung vorgeschlagen, wobei diese jedoch im Allgemeinen immer im ultravioletten Bereich liegen.

Beide Lichtquellen 11 und 12 sind vorzugsweise derart positioniert, dass sie das zu beobachtende Objekt aus der im Wesentlichen gleichen Richtung beleuchten, aus der dieses durch das optische System 15 der Kamera 100 erfasst wird. D.h., die Positionierung dieser Lichtquellen 11 und 12 wird im Bereich des Lichteintrittsfensters 9, bspw. verteilt an dessen Umfang erfolgen.

Die dritten Lichtquellen 13 schließlich sind diejenigen, die an den Enden der Arme 7 des Aufsatzes 5 angeordnet sind und seitlich Licht in den Zahn im Rahmen einer Transilluminationsaufnahme einkoppeln sollen. Wie erläutert sollen diese Lichtquellen 13 in erster Linie langwelliges Licht im Infrarotbereich, beispielsweise zwischen etwa 800 und 900nm, also z.B. Licht mit einer Wellenlänge von etwa 850nm, abgeben, da hier die innerhalb des Zahns resultierende Lichtstreuung besonders gut das Erkennen von Karies ermöglicht. Den Lichtquellen 13 können geeignete Lichtabgabe- bzw. Lichteinkoppelelemente zugeordnet sein, mit deren Hilfe die Lichteinstrahlung in den Zahn optimiert wird. Es kann sich hierbei um aus Silikon bestehende Elemente mit speziellen Strukturierungen handeln, die dann an der Außenfläche des zu untersuchenden Zahns anliegen.

Insgesamt weisen also die Beleuchtungsmittel 10 des Kamerahandstücks 10 drei verschiedene Lichtquellen 11, 12 und 13 auf, die je nach Beleuchtungsmodus bzw. Aufnahmemodus das zu beobachtende Objekt in unterschiedlicher Weise beleuchten. Hierbei ist anzumerken, dass insbesondere für die ersten beiden Lichtquellen 11 und 12 nicht zwingend eine physische Trennung der Leuchtmittel vorliegen muss. Da die spektrale Zusammensetzung des Lichts für die jeweils verschiedenen Aufnahmemodi allerdings nicht überlappt, werden letztendlich jeweils separate Leuchtmittel zum Einsatz kommen, die dann allerdings ggf. auf einer gemeinsamen Platine oder einem anderen gemeinsamen Träger angeordnet sind.

In Figur 3 ist weiterhin schematisch dargestellt, dass für den abnehmbar ausgestalteten Aufsatz 5 Stromversorgungsmittel 8 vorgesehen sein können, um die Leuchtmittel 13 des Aufsatzes 5 für die Transilluminationsaufnahme mit Energie zu versorgen. Bevorzugt kommen hierbei Stromversorgungsmittel zum Einsatz, die eine induktive, also eine kabellose Stromversorgung ermöglichen, was zu Vorteilen hinsichtlich der Möglichkeiten, das Kamerahandstück 100 zu reinigen, führt.

Ferner sind in Figur 3 die für die Bilderfassung verantwortlichen Komponenten erkennbar. Diese beinhalten zunächst einen digitalen Bildsensor 20, der innerhalb der Griffhülse 110 des Handstücks 100 angeordnet ist und mit einer nicht näher gezeigten Steuerelektronik verbunden ist. Als Bildsensor 20 kann beispielsweise ein Bayer-Sensor eingesetzt werden. Diesem Sensor 20 ist ein optisches System 15 vorgeschaltet, mit dessen Hilfe das über das Lichteintrittsfenster 9 einfallende Licht auf den Sensor 20 gerichtet wird. Dieses optische System 15 ist im vorliegenden Fall stark vereinfacht dargestellt, wobei es entsprechend der Darstellung eine dem Sensor 20 vorgeschaltete Fokussierlinse 17, ein am vorderen Ende des Handstücks 100 zur Lichtumlenkung vorgesehenes Umlenkprisma 18 sowie einen Filter 16 aufweist. Es handelt sich wie bereits erwähnt um eine stark vereinfachte Darstellung des optischen Systems 15, welches darüber hinaus gehend auch mehrere weitere Linsen und Blenden aufweisen wird, um eine kontrastreiche, scharfe Abbildung des Objekts auf den Sensor 20 zu gewährleisten. Auf die Darstellung dieser weiteren Komponenten wurde allerdings verzichtet, da die vorliegende Erfindung in erster Linie die Ausgestaltung des Filters 16 betrifft, was nachfolgend detailliert erläutert werden soll. Anzumerken ist allerdings, dass alternativ zu dem Umlenkprisma 18 selbstverständlich auch ein Umlenkspiegel zum Einsatz kommen könnte.

Der dem Sensor 20 vorgeschaltete, erfindungsgemäß ausgestaltete Filter 16 ist erforderlich, da sichergestellt werden muss, dass im Rahmen der unterschiedlichen Aufnahmemodi durch den Sensor 20 Licht nur in solchen Wellenlängenbereichen erfasst werden soll, die für den entsprechenden Aufnahmemodus relevant sind. Während es bislang bekannt war, für jeden Aufnahmemodus einen separaten Filter einzusetzen, wird erfindungsgemäß nunmehr vorgeschlagen, für alle drei Aufnahmemodi einen gemeinsamen Filter 16 zu verwenden, der nunmehr also fix innerhalb des Kamerahandstücks 100 angeordnet werden kann. Die Verwendung eines einzigen Filters für alle drei Aufnahmemodi wird durch dessen spezielle Ausgestaltung ermöglicht, die nachfolgend anhand von Figur 4 näher erläutert werden soll.

Figur 4 zeigt hierbei das Transmissionsspektrum 200 des erfindungsgemäßen Filters 16, wobei insbesondere erkennbar ist, dass dieser zwei Bereiche, sog. Bandpässe 201 und 202 aufweist, in denen der Filter 16 für Licht der entsprechenden Wellenlängen im Wesentlichen vollständig durchlässig ist, die durch einen Sperrbereich 203, in dem der Filter 204 für die entsprechenden Wellenlängen undurchlässig ist, getrennt sind. Der erste Bandpass 201 umfasst hierbei im Wesentlichen den Bereich des sichtbaren Lichts und erstreckt sich somit in etwa von 420nm bis 700nm. Der zweite Bandpass 202 hingegen liegt im Infrarotbereich des Lichts. Er ist im Vergleich zum ersten Bandpass 201 deutlich schmäler ausgeführt und erstreckt sich lediglich über den Bereich von 810 bis 890nm. Im dazwischenliegenden Sperrbereich 203 ist des Filter 200 wie bereits erwähnt undurchlässig. Auch im an den ersten Bandpass 201 angrenzenden kurzwelligeren Bereich 205 erfolgt wieder ein Blocken des Lichts bzw. allgemein elektromagnetischer Strahlung durch das Filter.

Diese spezielle Ausgestaltung des Transmissionsspektrums 200 des Filters 16 ermöglicht nunmehr, dass in Kombination mit einer entsprechend geeigneten Beleuchtung eine Aufnahme in jedem der drei Aufnahmemodi des Kamerahandstücks 100 durchgeführt werden kann, wobei nachfolgend die drei verschiedenen Aufnahmemodi einzeln besprochen werden sollen.

### a) Aufnahmemodus 1 - Weißlichtaufnahme

In diesem Modus soll wie bereits erwähnt ein Bild des zu beobachtenden Objekts im Wellenlängenbereich des sichtbaren Lichts erstellt werden. Die in diesem Fall verwendete Weißlichtquelle 11 beleuchtet also das Objekt, z.B. den Zahn wie oben beschrieben mit weißem Mischlicht, welches idealerweise den gesamten Bereich des sichtbaren Lichts abdeckt. Da für die Weißlichtaufnahme das von dem Objekt reflektierte Licht herangezogen wird und dieses durch die Reflexion in seiner Wellenlänge nicht verändert wird, wird durch den Bandpass 201 des Filters 16 eine qualitativ hochwertige Weißlichtaufnahme ermöglicht.

### b) Aufnahmemodus 2 - Fluoreszenzdiagnose

In diesem Fall wird der zu untersuchende Zahn mit einer Anregungsstrahlung 204 beleuchtet, die in etwa im Bereich von 400nm liegt. Trifft diese auf Karies bzw. kariöses Gewebe, so führen Fluoreszenzeffekte dazu, dass das Gewebe eine Fluoreszenzstrahlung abgibt, die im orange-roten Bereich des sichtbaren Lichts liegt. Durch Karies hervorgerufenes Fluoreszenzlicht kann durch den Sensor 20 erfasst werden, da dieses wiederum durch den ersten Bandpass 201 des Filters 16 durchgelassen wird. Gleichzeitig beeinträchtigt reflektiertes Anregungslicht die Aufnahme nicht, da dieses durch den an den Bandpass 201 angrenzenden Sperrbereich 205, der die unterhalb des sichtbaren Lichts liegenden kürzeren Wellenlängen betrifft, geblockt wird.

Anzumerken ist, dass in diesem Fall die Tatsache, dass sich der erste Bandpass 201 über einen deutlich größeren Wellenlängenbereich als das Spektrum der Fluoreszenzstrahlung erstreckt (diese ist auf einen verhältnismäßig schmalbandigen Bereich begrenzt), nicht zwingend negativ auf die Qualität der Aufnahme auswirkt, da der Zahn bzw. das Objekt nicht aktiv zusätzlich durch Weißlicht beleuchtet wird. Zwar wird aus der Umgebung einfallendes Licht möglicherweise durch die Zahnoberfläche reflektiert und ebenfalls durch den Sensor 20 erfasst werden, diese geringe Menge an sichtbarem Licht kann allerdings sogar von Vorteil sein, da in diesem Fall dann auch zumindest die Kontur des beobachteten Zahns leicht erkennbar ist, wobei allerdings von Karies betroffene Bereiche durch die Fluoreszenzstrahlung deutlich hervorgehoben werden.

In diesem Aufnahmemodus kann ferner eine zusätzliche Verbesserung der Bildqualität erzielt werden, indem die Empfindlichkeitswerte für diejenigen Farbpunkte (Pixel), welche den Blauanteil im CCD Chip des Bildsensors 20 detektieren, verringert wird. Die kurzwellige Anregungsstrahlung 204, welche trotz des Filters 16 den Bildsensor 20 erreicht, wird durch diese Maßnahme zusätzlich blockiert.

### c) Aufnahmemodus 3 - Transillumination

In diesem Fall wird Licht etwa im Wellenlängenbereich zwischen 810nm und 890nm in den Zahn eingekoppelt und durch diesen gestreut. Hierbei erfolgt keine Wellenlängenveränderung des Lichts, sodass auch hier die Ausgestaltung des Filters 16, insbesondere der Bandpass 202 dazu beiträgt, dass durch den Sensor 20 Licht geeigneter Wellenlänge für die Transilluminationsaufnahme erfasst wird. Da Licht mit Wellenlängen aus dem umgebenden Bereich durch den Filter 16 wiederum geblockt wird, wird die Aufnahme auch nicht durch andere Lichtanteile negativ beeinträchtigt.

Zwar könnte auch bei diesem Aufnahmemodus Licht aus dem sichtbaren Bereich durchaus aufgrund des ersten Bandpasses 201 auf den Sensor 20 treffen, der Anteil an Umgebungslicht ist in diesem Fall allerdings verhältnismäßig gering, da bei der Transilluminationsaufnahme aufgrund der hierzu erforderlichen speziellen Positionierung des Kamerahandstücks 100 bzgl. des Zahns nur sehr wenig Umgebungslicht überhaupt auf den Zahn fallen kann. Insbesondere der Aufsatz 5 trägt dazu bei, dass nahezu kaum Umgebungslicht auf den Zahn fallen wird. Die weiteren Lichtquellen 11 und 12 für die Weißlichtaufnahme und die Fluoreszenzdiagnose sind selbstverständlich in diesem Fall deaktiviert.

Aus den obigen Schilderungen ergibt sich also, dass der erfindungsgemäß ausgestaltete Filter perfekt dazu geeignet ist, für alle drei verschiedenen Aufnahmemodi der Kamera 100 genutzt zu werden. Es ist lediglich erforderlich, dass für einen entsprechenden Aufnahmemodus die zugehörige Lichtquelle aktiviert ist.

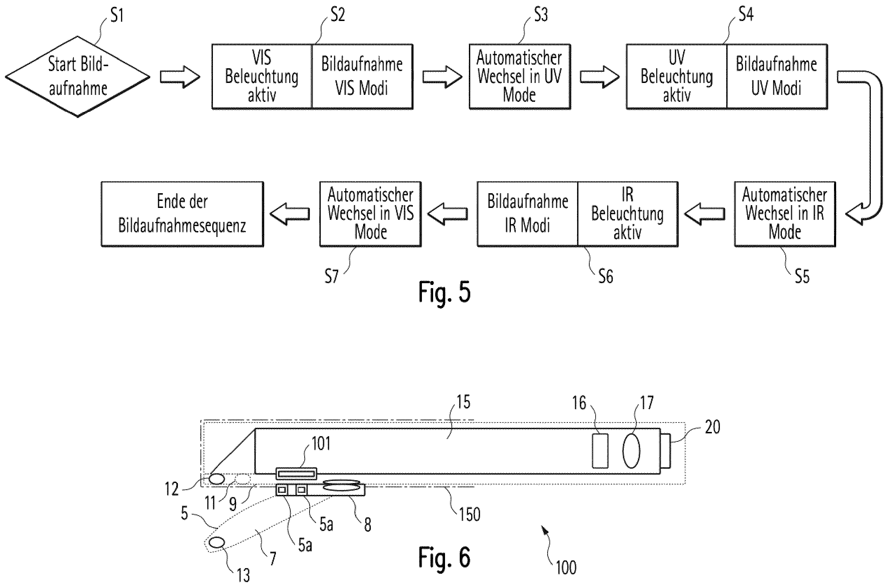

Es ergibt sich dann für den Fall, dass eine Bildsequenz bestehend aus drei Aufnahmen in verschiedenen Aufnahmemodi erstellt werden soll, ein Ablauf, wie er schematisch in Figur 5 dargestellt ist. Durch entsprechendes Aktivieren der Kamera 100, bspw. über einen Betätigungsknopf im Bereich der Griffhülse 110 wird der Bildaufnahmemodus in Schritt S1 gestartet. In einem darauffolgenden Schritt wird dann zunächst die Lichtquelle 11 für die Weißlichtaufnahme aktiviert und ein Bild durch den Sensor 20 erfasst. Anschließend erfolgt in Schritt 3 ein Wechsel in den UV-Aufnahmemodus, also die Fluoreszenzdiagnose. Wiederum wird hier zunächst die entsprechende Beleuchtung, also die Lichtquelle 12 aktiviert und ein Bild durch den Sensor aufgenommen (Schritt S4). Nachdem in Schritt S5 in den Transilluminationsaufnahmemodus gewechselt wird, wird die entsprechende Beleuchtung durch die Lichtquellen 13 aktiviert und eine Aufnahme erstellt (Schritt S6), wobei dann zum Abschluss der Sequenz wieder in den Weißlichtaufnahmemodus gewechselt wird (Schritt S7), wodurch die Aufnahmesequenz beendet wird.

Die Tatsache, dass für einen Wechsel zwischen den verschiedenen Aufnahmemodi lediglich erforderlich ist, die zugehörige Lichtquelle zu aktivieren, führt dazu, dass nahezu ohne zeitliche Verzögerung Aufnahmen in drei verschiedenen Aufnahmemodi nacheinander erstellt werden können. Wird davon ausgegangen, dass der Zahnarzt in diesem sehr kurzen Zeitraum die Kamera 100 nicht bzw. nur unwesentlich bewegt, führt dies dazu, dass alle drei Aufnahmen den identischen Bildausschnitt beinhalten. Ohne vorherige Bildanalyse können dann die entsprechenden Aufnahmen überlagert werden oder ggf. auch nebeneinander auf einem Bildschirm dargestellt werden. In jedem Fall wird hierdurch die Möglichkeit, die Aufnahmen miteinander zu vergleichen und/oder zu kombinieren, deutlich verbessert.

Abschließend soll anhand von Figur 6 noch ein alternativer Aufbau des erfindungsgemäßen Kamerahandstücks 100 erläutert werden. Im Vergleich zu der Darstellung von Figur 3 werden hierbei für identische Komponenten identische Bezugszeichen verwendet. Es ist also erkennbar, dass bei dieser Variante zusätzlich noch im vorderen Endbereich der Griffhülse 110 Sensoren 101 angeordnet sind, die mit entsprechenden Codierelementen 5a am abnehmbaren Aufsatz 5 zusammenwirken. Die Sensoren 101 dienen dazu, das Vorhandensein eines Aufsatzes 5 grundsätzlich und ggf. auch dessen Typ zu erkennen. Bei den Codierelementen kann es sich dementsprechend um geeignete elektronische Komponenten wie Induktivitäten oder Kapazitäten handeln. Auch die Verwendung von sog. RFID-Tags wäre denkbar. Besonders bevorzugt erfolgt die Erkennung allerdings mittels Hall Sensoren 101. Diese Sensoren 101 sind an der Griffhülse 110 der Kamera 100 positioniert und detektieren in den Aufsatz 5 integrierte Magnete 5a sowie deren Orientierung (Nord/Süd). Pro Aufsatz 5 können hierbei mindestens zwei Magnete vorgesehen sein.

Das grundsätzliche Erkennen des Vorhandenseins eines Aufsatzes 5 ist bspw. insofern von Vorteil, als für den Fall, dass kein Aufsatz 5 vorhanden ist, die anhand von Figur 5 erläuterte Aufnahmesequenz automatisch auf Aufnahmen im sichtbaren Weißlichtbereich sowie auf Aufnahmen im Rahmen der Fluoreszenzdiagnose beschränkt werden kann. Erst für den Fall, dass das Vorhandensein des Aufsatzes 5 erkannt wird, kann dann auch die Transilluminationsaufnahme der Aufnahmesequenz hinzugefügt und eine entsprechende Stromversorgung der Lichtquellen 13 des Aufsatzes vorgenommen werden. Ferner ist es ggf. erforderlich, je nach Aufsatz 5 unterschiedliche Parameter in der Kamera 100 und in der Energieübertragung einzustellen. Dies wird durch die bevorzugte kontaktlose Erkennung Aufsätze 5 deutlich vereinfacht.

Ferner ist in Figur 6 schematisch eine sog. Hygieneschutzhülle 150 gezeigt, welche als Wegwerfprodukt vor Verwendung der Kamera 100 über diese übergestülpt wird. Diese Hülle liegt an der Griffhülse 110 außen an und ist zumindest im Bereich des Sichtfensters 9 lichtdurchlässig bzw. klar ausgestaltet, um die Bilderfassung nicht negativ zu beeinträchtigen. Kommt ein Aufsatz 5 zum Einsatz, so wird dieser auf die Kamera 100 mit aufgestülpter Hygieneschutzhülle 150 gesteckt. Die drahtlose Identifizierung der Aufsätze 5 sowie die drahtlose Energieversorgung bringt hier besondere Vorteile mit sich, da die Hygieneschutzhülle 150 in diesem Fall tatsächlich vollständig geschlossen ausgeführt sein kann und dementsprechend das Kamerahandstück 100 optimal geschützt werden kann.

Insgesamt wird durch die Erfindung also eine Kamera zu zahnärztlichen Zwecken zur Verfügung gestellt, welche hervorragende Aufnahmeeigenschaften aufweist und insbesondere die Möglichkeit eröffnet, Aufnahmen im Rahmen unterschiedlicher Aufnahmemodi zu erstellen.

## Patentansprüche

1. Zahnärztliches Kamerahandstück (100) zum Erstellen intraoraler Aufnahmen, aufweisend:
• Beleuchtungsmittel (10) zum Beleuchten eines vor einem Eintrittsfenster (9) des Kamerahandstücks (100) befindlichen Objekts,
• ein optisches System (15) zum Abbilden des Objekts auf einen Bildsensor (20),
• einen digitalen Bildsensor (20) zum Erfassen des mit Hilfe des optischen Systems (15) auf den Sensor (20) abgebildeten Objekts,
wobei die Beleuchtungsmittel (10) dazu ausgebildet sind, das vor dem Eintrittsfenster (9) des Kamerahandstücks (100) befindliche Objekt entsprechend drei verschiedener Aufnahmemodi zu beleuchten, wobei die spektrale Zusammensetzung des von den Beleuchtungsmitteln (10) abgegebenen Lichts für jeden Aufnahmemodus unterschiedlich ist, und
wobei das optisches System (15) einen dem Bildsensor (20) vorgeschalteten und für alle drei Aufnahmemodi gemeinsam genutzten Filter (16) aufweist, dessen Transmissionsspektrum (200) einen ersten Bandpass aufweist (201), der im Wesentlichen dem Wellenlängenbereich des sichtbaren Lichts entspricht, sowie einen zweiten Bandpass (202), dessen Wellenlängen im Infrarotbereich liegen, wobei der erste Bandpass (201) von dem zweiten Bandpass (202) durch einen Sperrbereich (203) getrennt ist, in dem der Filter (16) im Wesentlichen dessen Wellenlängenbereich sperrt,
wobei Beleuchtungsmittel (10) dazu ausgebildet sind:
• in einem Weißlicht-Aufnahmemodus das Objekt mit sichtbarem Licht zu beleuchten,
• in einem Fluoreszenzdiagnose-Modus das Objekt mit einer Anregungsstrahlung zu beleuchten, deren spektrale Zusammensetzung im Wesentlichen unterhalb des sichtbaren Lichts liegt, wobei der Wellenlängenbereich der Anregungsstrahlung (204) von dem ersten Bandpass (201) des Filters (16) durch einen zweiten Sperrbereich (205) getrennt ist, in dem der Filter (16) im Wesentlichen dessen Wellenlängenbereich sperrt, und
• in einem Transilluminations-Modus das Objekt mit Licht eines Wellenlängenbereichs zu beleuchten, der im Wesentlichen in dem zweiten Bandpass (202) des Filters (16) liegt.

2. Zahnärztliches Kamerahandstück nach Anspruch 1,
**dadurch gekennzeichnet,**
**dass** der für alle drei Aufnahmemodi gemeinsam genutzte Filter (16) fix innerhalb des Kamerahandstücks (100) angeordnet ist.

3. Zahnärztliches Kamerahandstück nach Anspruch 1 oder 2,
**dadurch gekennzeichnet,**
**dass** der zweite Bandpass (202) im Vergleich zum ersten Bandpass (201) deutlich schmäler ausgeführt ist,
wobei vorzugsweise der erste Bandpass (201) sich von 420nm bis 700nm erstreckt und der zweite Bandpass (202) sich von 810nm bis 890nm erstreckt.

4. Zahnärztliches Kamerahandstück nach einem der Ansprüche 1 bis 3,
**dadurch gekennzeichnet,**
**dass** die Beleuchtungsmittel (10) eine erste Lichtquelle (11) aufweisen, welche dazu ausgebildet ist, in dem Weißlicht-Aufnahmemodus das Objekt mit dem sichtbarem Licht, insbesondere mit Weißlicht zu beleuchten.

5. Zahnärztliches Kamerahandstück nach einem der Ansprüche 1 bis 4,
**dadurch gekennzeichnet,**
**dass** die Beleuchtungsmittel (10) eine zweite Lichtquelle (12) aufweisen, welche dazu ausgebildet ist, in dem Fluoreszenzdiagnose-Modus das Objekt mit der Anregungsstrahlung zu beleuchten.

6. Zahnärztliches Kamerahandstück nach Anspruch 4 und Anspruch 5,
**dadurch gekennzeichnet,**
**dass** die erste und zweite Lichtquelle (11, 12) derart positioniert sind, dass sie das zu beobachtende Objekt aus der im Wesentlichen gleichen Richtung beleuchten, aus der dieses durch das optische System des Kamerahandstücks (100) erfasst wird.

7. Zahnärztliches Kamerahandstück nach Anspruch 4 und 5 oder nach Anspruch 6,
**dadurch gekennzeichnet,**
**dass** jeweils separate Leuchtmittel für die ersten beiden Lichtquellen (11, 12) zum Einsatz kommen, die auf einer gemeinsamen Platine oder einem anderen gemeinsamen Träger angeordnet sind.

8. Zahnärztliches Kamerahandstück nach einem der Ansprüche 5 bis 7,
**dadurch gekennzeichnet,**
**dass** dieses dazu ausgebildet ist, die Abgabe der Anregungsstrahlung zeitlich zu modulieren.

9. Zahnärztliches Kamerahandstück nach einem der Ansprüche 5 bis 8,
**dadurch kennzeichnet,**
**dass** in dem Fluoreszenzdiagnose-Modus die Empfindlichkeitswerte für diejenigen Pixel des Bildsensors (20), welche den Blauanteil detektieren, verringert wird.

10. Zahnärztliches Kamerahandstück nach einem der vorherigen Ansprüche,
**dadurch gekennzeichnet,**
**dass** die Beleuchtungsmittel (10) eine dritte Lichtquelle (13) aufweisen, welche dazu ausgebildet ist, in dem Transilluminations-Modus das Objekt mit Licht des Wellenlängenbereichs zu beleuchten, der im Wesentlichen in dem zweiten Bandpass (202) des Filters (16) liegt.

11. Zahnärztliches Kamerahandstück nach Anspruch 10,
**dadurch gekennzeichnet,**
**dass** die dritte Lichtquelle (13) Bestandteil eines Aufsatzes (5) ist, der lösbar am vorderen Ende des Kamerahandstücks (100) angeordnet ist, wobei das Objekt in einer Richtung beleuchtet wird, die im Wesentlichen senkrecht zur Beleuchtungsrichtung für den Weißlicht-Aufnahmemodus oder den Fluoreszenzdiagnose-Modus ist.

12. Zahnärztliches Kamerahandstück nach Anspruch 11,
**dadurch gekennzeichnet,**
**dass** dieses einen Sensor (101) zum Erkennen der Anordnung eines Aufsatzes (5) an dem Kamerahandstück (100) sowie ggf. des Typs des Aufsatzes (5) aufweist.

13. Zahnärztliches Kamerahandstück nach Anspruch 11 oder 12,
**dadurch gekennzeichnet,**
**dass** dieses Mittel zur Energieversorgung (8) der in dem Aufsatz befindlichen Lichtquelle (13) aufweist, wobei die Mittel zur Energieversorgung (8) insbesondere für eine induktive Energieversorgung ausgebildet sind.

14. Zahnärztliches Kamerahandstück nach einem der vorherigen Ansprüche,
**dadurch gekennzeichnet,**
**dass** dieses eine Hygieneschutzhülle (150) aufweist, welche über das Kamerahandstück (100) überstülpbar ist und zumindest im Bereich des Eintrittsfensters (9) lichtdurchlässig ausgebildet ist.

15. Zahnärztliches Kamerahandstück nach einem der vorherigen Ansprüche,
**dadurch gekennzeichnet,**
**dass** dieses dazu ausgebildet ist, die Beleuchtungsmittel (10) entsprechend einer vorgegebenen Sequenz zu betreiben, welche der alternierenden Nutzung der verschiedenen Aufnahmemodi entspricht.

16. Zahnärztliches Kamerahandstück nach Anspruch 15,
**dadurch gekennzeichnet,**
**dass** eine Aufnahmesequenz der Aufnahme jeweils eines Bildes ein jedem der drei Aufnahmemodi entspricht.

17. Zahnärztliches Kamerahandstück nach einem der vorherigen Ansprüche,
**dadurch gekennzeichnet,**
**dass** dieses eine längliche und rohrartig ausgestaltete Griffhülse aufweist, die an ihrem rückseitigen Ende über ein Anschlusskabel (115) mit einem externen Gerät verbindbar ist, wobei die Verbindung insbesondere über einen USB-Anschluss (116) erfolgt.

18. Zahnärztliches Kamerahandstück nach Anspruch 17,
**dadurch gekennzeichnet,**
**dass** die Griffhülse am vorderen Ende ein Lichteintrittsfenster aufweist,
wobei das optische System (15) zum Abbilden des Objekts auf den Bildsensor (20) sowie der Bildsensor (20) innerhalb der Griffhülse angeordnet sind und das optische System (15) das über das Lichteintrittsfenster eintretende Licht auf den Bildsensor (20) lenkt,
und wobei vorzugsweise eine Flächennormale des Lichteintrittsfensters im Wesentlichen senkrecht zur Längsachse der Griffhülse ausgerichtet ist und im vorderen Ende der Griffhülse ein Umlenkelement, beispielsweise ein Spiegel oder ein Prisma angeordnet ist.

## Claims

1. Dental camera handpiece (100) for creating intraoral recordings, comprising:
• lighting means (10) for illuminating an object situated in front of an entrance window (9) of the camera handpiece (100),
• an optical system (15) for imaging the object onto an image sensor (20),
• a digital image sensor (20) for capturing the object imaged on the sensor (20) with the aid of the optical system (15),
the lighting means (10) being designed to illuminate the object situated in front of the entrance window (9) of the camera handpiece (100) in accordance with three different recording modes, with the spectral composition of the light emitted by the lighting means (10) being different for each recording mode, and
the optical system (15) comprising a filter (16) commonly used for all three recording modes which is disposed upstream of the image sensor (20), the transmission spectrum (200) of said filter having a first bandpass (201) which substantially corresponds to the wavelength range of visible light and a second bandpass (202) whose wavelengths are in the infrared range, the first bandpass (201) being separated from the second bandpass (202) by a blocking range (203) in which the filter (16) substantially blocks the wavelength range thereof,
with the lighting means (10) being designed:
• to illuminate the object with visible light in a white-light recording mode,
• to illuminate the object with excitation radiation in a fluorescence diagnosis mode, the spectral composition of said excitation radiation being substantially located below the visible light, with the wavelength range of the excitation radiation (204) being separated from the first bandpass (201) of the filter (16) by a second blocking range (205) in which the filter (16) substantially blocks the wavelength range thereof, and
• to illuminate the object with light in a transillumination mode, the wavelength range of said light being substantially located within the second bandpass (202) of the filter (16).

2. Dental camera handpiece according to Claim 1,
**characterized**
**in that** the filter (16) commonly used for all three recording modes is fixedly arranged within the camera handpiece (100).

3. Dental camera handpiece according to Claim 1 or 2,
**characterized**
**in that** the second bandpass (202) has a significantly narrower form in comparison with the first bandpass (201),
the first bandpass (201) preferably extending from 420 nm to 700 nm and the second bandpass (202) preferably extending from 810 nm to 890 nm.

4. Dental camera handpiece according to any one of Claims 1 to 3,
**characterized**
**in that** the lighting means (10) comprise a first light source (11) which is designed to illuminate the object with the visible light, in particular with white light, in the white-light recording mode.

5. Dental camera handpiece according to any one of Claims 1 to 4,
**characterized**
**in that** the lighting means (10) comprise a second light source (12) which is designed to illuminate the object with the excitation radiation in the fluorescence diagnosis mode.

6. Dental camera handpiece according to Claim 4 and Claim 5,
**characterized**
**in that** the first and the second light sources (11, 12) are positioned such that they illuminate the object to be observed from substantially the same direction as from which the said object to be observed is captured by the optical system of the camera handpiece (100).

7. Dental camera handpiece according to Claims 4 and 5 or according to Claim 6,
**characterized**
**in that** separate illuminants are used in each case for the first two light sources (11, 12), said separate illuminants being arranged on a common printed circuit board or on any other common carrier.

8. Dental camera handpiece according to any one of Claims 5 to 7,
**characterized**
**in that** said dental camera handpiece is designed to modulate the emission of the excitation radiation over time.

9. Dental camera handpiece according to any one of Claims 5 to 8,
**characterized**
**in that** the sensitivity values for those pixels of the image sensor (20) which detect the blue component are reduced in the fluorescence diagnosis mode.

10. Dental camera handpiece according to any one of the preceding claims,
**characterized**
**in that** the lighting means (10) comprise a third light source (13) which is designed to illuminate the object in the transillumination mode with light of the wavelength range substantially located within the second bandpass (202) of the filter (16).

11. Dental camera handpiece according to Claim 10,
**characterized**
**in that** the third light source (13) is a constituent part of an attachment (5) detachably arranged at the front end of the camera handpiece (100), the object being illuminated in a direction which is substantially perpendicular to the illumination direction for the white-light recording mode or the fluorescence diagnosis mode.

12. Dental camera handpiece according to Claim 11,
**characterized**
**in that** said dental camera handpiece comprises a sensor (101) for recognizing the arrangement of an attachment (5) on the camera handpiece (100) and optionally for recognizing the type of the attachment (5).

13. Dental camera handpiece according to Claim 11 or 12,
**characterized**
**in that** said dental camera handpiece comprises power supply means (8) for the light source (13) situated in the attachment, the power supply means (8) being designed for an inductive power supply in particular.

14. Dental camera handpiece according to any one of the preceding claims,
**characterized**
**in that** said dental camera handpiece comprises a hygienic protective sleeve (150), which is able to be pulled over the camera handpiece (100) and which has a light-transmissive design, at least in the region of the entrance window (9).

15. Dental camera handpiece according to any one of the preceding claims,
**characterized**
**in that** said dental camera handpiece is designed to operate the lighting means (10) in accordance with a specified sequence, which corresponds to the alternate use of the various recording modes.

16. Dental camera handpiece according to Claim 15,
**characterized**
**in that** a recording sequence of the recording corresponds to a respective image in each of the three recording modes.

17. Dental camera handpiece according to any one of the preceding claims,
**characterized**
**in that** said dental camera handpiece has an elongate grip sleeve with a tubular design, which at its rear end is connectable to an external device by way of a connection cable (115), the connection being implemented via a USB connection (116) in particular.

18. Dental camera handpiece according to Claim 17,
**characterized**
**in that** the grip sleeve has a light-entrance window at the front end,
the optical system (15) for imaging the object on the image sensor (20) and said image sensor (20) being arranged within the grip sleeve and the optical system (15) steering the light entering via the light-entrance window onto the image sensor (20),
and a surface normal of the light-entrance window preferably being aligned substantially perpendicular to the longitudinal axis of the grip sleeve and a deflection element, for example a mirror or a prism, being arranged in the front end of the grip sleeve.

## Revendications

1. Pièce à main de caméra dentaire (100) pour réaliser des enregistrements intrabuccaux, présentant :
- des moyens d'éclairage (10) pour éclairer un objet se trouvant devant une fenêtre d'entrée (9) de la pièce à main de caméra (100),
- un système optique (15) pour reproduire l'objet sur un capteur d'image (20),
- un capteur d'image (20) numérique pour détecter l'objet reproduit sur le capteur (20) à l'aide du système optique (15),
dans lequel les moyens d'éclairage (10) sont réalisés pour éclairer des objets se trouvant devant la fenêtre d'entrée (9) de la pièce à main de caméra (100) conformément à trois différents modes d'enregistrement, dans laquelle la composition spectrale de la lumière émise par les moyens d'éclairage (10) est différente pour chaque mode d'enregistrement, et
dans laquelle le système optique (15) présente un filtre (16) branché en amont du capteur d'image (20) et utilisé conjointement pour tous les trois modes d'enregistrement, dont le spectre de transmission (200) présente un premier filtre passe-bande (201) qui correspond sensiblement à la plage de longueurs d'onde de la lumière visible, ainsi qu'un deuxième filtre passe-bande (202) dont les longueurs d'onde se situent dans la plage de l'infrarouge, dans laquelle le premier filtre passe-bande (201) est séparé du deuxième filtre passe-bande (202) par une zone de blocage (203), dans laquelle le filtre (16) bloque sensiblement sa plage de longueurs d'onde,
dans laquelle des moyens d'éclairage (10) sont réalisés :
- pour éclairer l'objet avec de la lumière visible dans un mode d'enregistrement à la lumière blanche,
- pour éclairer, dans un mode de diagnostic de fluorescence, l'objet avec un rayonnement d'excitation, dont la composition spectrale se situe sensiblement sous la lumière visible, dans laquelle la plage de longueurs d'onde du rayonnement d'excitation (204) est séparée du premier filtre passe-bande (201) du filtre (16) par une deuxième plage de blocage (205), dans laquelle le filtre (16) bloque sensiblement sa plage de longueurs d'onde, et
- pour éclairer, dans un mode de transillumination, l'objet avec de la lumière d'une plage de longueurs d'onde, qui se situe sensiblement dans le deuxième filtre passe-bande (202) du filtre (16).

2. Pièce à main de caméra dentaire selon la revendication 1,
**caractérisée en ce**
**que** le filtre (16) utilisé conjointement pour la totalité des trois modes d'enregistrement est disposé de manière fixe à l'intérieur de la pièce à main de caméra (100).

3. Pièce à main de caméra dentaire selon la revendication 1 ou 2,
**caractérisée en ce**
**que** le deuxième filtre passe-bande (202) est réalisé de manière nettement plus étroite en comparaison avec le premier filtre passe-bande (201),
dans laquelle de préférence le premier filtre passe-bande (201) s'étend de 420 nm à 700 nm et le deuxième filtre passe-bande (202) s'étend de 810 nm à 890 nm.

4. Pièce à main de caméra dentaire selon l'une quelconque des revendications 1 à 3,
**caractérisée en ce**
**que** les moyens d'éclairage (10) présentent une première source de lumière (11), laquelle est réalisée pour éclairer l'objet avec la lumière visible, en particulier avec de la lumière blanche, dans le mode d'enregistrement à la lumière blanche.

5. Pièce à main de caméra dentaire selon l'une quelconque des revendications 1 à 4,
**caractérisée en ce**
**que** les moyens d'éclairage (10) présentent une deuxième source de lumière (12), laquelle est réalisée pour éclairer l'objet avec le rayonnement d'excitation dans le mode de diagnostic de fluorescence.

6. Pièce à main de caméra dentaire selon la revendication 4 et la revendication 5,
**caractérisée en ce**
**que** la première et la deuxième source de lumière (11, 12) sont positionnées de telle manière qu'elles éclairent l'objet à observer depuis la direction sensiblement identique, depuis laquelle celui-ci est détecté par le système optique de la pièce à main de caméra (100).

7. Pièce à main de caméra dentaire selon la revendication 4 et 5 ou selon la revendication 6,
**caractérisée en ce**
**que** sont mis en œuvre des moyens lumineux respectivement séparés pour les deux premières sources de lumière (11, 12), qui sont disposés sur une platine commune ou sur un autre support commun.

8. Pièce à main de caméra dentaire selon l'une quelconque des revendications 5 à 7,
**caractérisée en ce**
**que** celle-ci est réalisée pour moduler dans le temps l'émission du rayonnement d'excitation.

9. Pièce à main de caméra dentaire selon l'une quelconque des revendications 5 à 8,
**caractérisée en ce**
**que** dans le mode de diagnostic de fluorescence, les valeurs de sensibilité pour les pixels du capteur d'image (20), qui précisément détectent la proportion de bleu, sont réduites.

10. Pièce à main de caméra dentaire selon l'une quelconque des revendications précédentes,
**caractérisée en ce**
**que** les moyens d'éclairage (10) présentent une troisième source de lumière (13), laquelle est réalisée pour éclairer, dans le mode de transillumination, l'objet avec de la lumière de la plage de longueurs d'onde, qui se situe sensiblement dans le deuxième filtre passe-bande (202) du filtre (16).

11. Pièce à main de caméra dentaire selon la revendication 10,
**caractérisée en ce**
**que** la troisième source de lumière (13) fait partie intégrante d'un embout (5), qui est disposé de manière amovible sur l'extrémité avant de la pièce à main de caméra (100), dans laquelle l'objet est éclairé dans une direction, qui est sensiblement perpendiculaire par rapport à la direction d'éclairage pour le mode d'enregistrement à la lumière blanche ou le mode de diagnostic de fluorescence.

12. Pièce à main de caméra dentaire selon la revendication 11,
**caractérisée en ce**
**que** celle-ci présente un capteur (101) pour identifier l'agencement d'un embout (5) sur la pièce à main de caméra (100) et éventuellement le type de l'embout (5).

13. Pièce à main de caméra dentaire selon la revendication 11 ou 12,
**caractérisée en ce**
**que** celle-ci présente des moyens pour l'alimentation en énergie (8) de la source de lumière (13) se trouvant dans l'embout, dans laquelle les moyens pour l'alimentation en énergie (8) sont réalisés en particulier pour une alimentation en énergie inductive.

14. Pièce à main de caméra dentaire selon l'une quelconque des revendications précédentes,
**caractérisée en ce**
**que** celle-ci présente une enveloppe de protection hygiénique (150), laquelle peut être retroussée par-dessus la pièce à main de caméra (100) et est réalisée de manière translucide au moins dans la zone de la fenêtre d'entrée (9).

15. Pièce à main de caméra dentaire selon l'une quelconque des revendications précédentes,
**caractérisée en ce**
**que** celle-ci est réalisée pour faire fonctionner les moyens d'éclairage (10) conformément à une séquence prédéfinie, laquelle correspond à l'utilisation en alternance des différents modes d'enregistrement.

16. Pièce à main de caméra dentaire selon la revendication 15,
**caractérisée en ce**
**qu'**une séquence d'enregistrement correspond à l'enregistrement de respectivement une image d'un quelconque des trois modes d'enregistrement.

17. Pièce à main de caméra dentaire selon l'une quelconque des revendications précédentes,
**caractérisée en ce**
**que** celle-ci présente un manchon de préhension allongé et de configuration tubulaire, qui peut être relié sur son extrémité côté arrière par l'intermédiaire d'un câble de raccordement (115) à un appareil externe, dans laquelle la liaison est effectuée en particulier par l'intermédiaire d'une borne USB (116).

18. Pièce à main de caméra dentaire selon la revendication 17,
**caractérisée en ce**
**que** le manchon de préhension présente sur l'extrémité avant une fenêtre d'entrée de lumière, dans laquelle le système optique (15) pour reproduire l'objet est disposé sur le capteur d'image (20) et le capteur d'image (20) est disposé à l'intérieur du manchon de préhension, et le système optique (15) dirige la lumière entrant par l'intermédiaire de la fenêtre d'entrée de lumière sur le capteur d'image (20),
et dans laquelle de préférence une normale de surface de la fenêtre d'entrée de lumière est orientée de manière sensiblement perpendiculaire par rapport à l'axe longitudinal du manchon de préhension, et un élément de renvoi, en particulier un miroir ou un prisme, est disposé dans l'extrémité avant du manchon de préhension.
